# DEMANDE DE BREVET EUROPEEN

(11) **EP 4 056 103 A1**
(43) Date de publication de la demande: **14.09.2022**
(21) Numéro de dépôt: 21305272.3
(22) Date de dépôt: 08.03.2021
(51) Int. Cl.: A61B 5/00, A61B 5/11, A61B 5/113

(54) **SYSTÈME DE CARACTÉRISATION DE L'ACTIVITÉ PHARYNGOLARYNGÉE COMPRENANT PLUSIEURS CAPTEURS DE MÊME TYPE ET PROCÉDÉ ASSOCIÉ**

(71) Demandeur: Swallis Médical, 67000 Strasbourg (FR)
(72) Inventeur: PERRIN, Nicolas, 67120 Kolbsheim (FR); NEVEU, Fabrice, 34000 Montpellier (FR); LE COZ, Maxime, 82170 Grisolles (FR); NICOLINI, Linda, 31200 Toulouse (FR)
(74) Mandataire: Cabinet Camus Lebkiri

(57) **Abrégé**

Un aspect de l'invention concerne un système de caractérisation de l'activité pharyngolaryngée d'un utilisateur, le système comprenant au moins :
- un dispositif de captation comprenant une pluralité de capteurs d'activité pharyngolaryngée, la pluralité de capteurs comprenant au moins deux capteurs d'activité pharyngolaryngée de même type placés selon une configuration spatiale prédéterminée, chaque capteur étant configuré pour mesurer au moins un signal d'activité pharyngolaryngée de l'utilisateur lors d'au moins une activité pharyngolaryngée de l'utilisateur,
- au moins un processeur configuré pour réaliser une caractérisation de la au moins une activité pharyngolaryngée de l'utilisateur, la caractérisation étant basée sur chacun des signaux d'activité pharyngolaryngée provenant de chacun des capteurs de la pluralité de capteurs d'activité pharyngolaryngée.

## Description

### DOMAINE TECHNIQUE DE L'INVENTION

Le domaine technique de l'invention est celui de la caractérisation d'activités pharyngolaryngées.

La présente invention concerne un système de caractérisation de l'activité pharyngolaryngée et en particulier un système comprenant une pluralité de capteurs de même type dans une configuration spatiale prédéterminée et un modèle de caractérisation automatique permettant la caractérisation de signaux issus de l'ensemble des capteurs, les capteurs étant par exemple de type accéléromètres et/ou microphones.

### ARRIERE-PLAN TECHNOLOGIQUE DE L'INVENTION

Les patients atteints de troubles de la déglutition, aussi appelés « dysphagie », présentent des difficultés à avaler certains types et/ou textures d'aliments, et des risques de fausses-routes. Concernant les fausses-routes, on parle de pénétration si l'aliment entre dans le larynx mais reste au-dessus de la glotte, des cordes vocales, et d'aspiration s'il passe les cordes vocales. L'aspiration entraîne une des réponses corporelles, normalement une toux, mais l'aspiration peut être sans toux. Elle est dans ce cas appelée « silencieuse ».

La dysphagie peut par exemple apparaître chez des patients après un accident vasculaire cérébral (« AVC »), un traumatisme crânio-cérébral (« TCC »), une Sclérose Latérale Amiotrophique ou chez les patients atteints de la maladie d'Alzheimer ou de maladie neurodégénérative.

Afin de caractériser la gravité de la dysphagie d'un patient, par exemple pour évaluer le risque de fausse route, le type, la cause et/ou le niveau de gravité de la dysphagie, de nombreuses méthodes d'examen ont été développées.

L'étude de l'accélérométrie de la déglutition a notamment été développée face à un besoin de méthodes non-invasives d'évaluation de la dysphagie, particulièrement grâce à la miniaturisation et à l'amélioration de la précision des accéléromètres électroniques. Des dispositifs colliers comprenant un accéléromètre ont été développés pour l'acquisition de signaux d'accélérométrie de déglutition au niveau du larynx. En effet, une déglutition peut être divisée en trois phases :
- la phase orale de la déglutition qui est volontaire et comprend le mouvement postérieur de la langue et de l'os hyoïde,
- la phase laryngo-pharyngée qui est automatique et réflexe et comprend le mouvement laryngé, l'élévation de l'os hyoïde, la fermeture de l'épiglotte et le passage du bolus vers l'orifice œsophagien, et
- la phase oesophagienne qui est réflexe et comprend la contraction péristaltique, le repositionnement de l'os hyoïde et du larynx et la réouverture de l'épiglotte.

La captation de ces signaux permet leur traitement par ordinateur et leur caractérisation lors de ces trois phases de la déglutition, et des techniques de classification automatique ont ainsi été appliquées à ces signaux dans l'état de la technique pour la détection de dysphagies, d'aspirations, de fausses-routes silencieuses et/ou pour l'évaluation de la gravité de la dysphagie.

De tels dispositifs colliers peuvent en outre comprendre un capteur de son laryngé pour la mesure du son de déglutition, par exemple afin de caractériser les sons de respiration, de toux et les sons liés à la voix du patient. Le microphone peut aussi permettre la détection de la déglutition, par exemple en détectant un ensemble de sons liés à l'ascension laryngée correspondant à l'ascension du larynx quand le bolus est localisé dans l'oropharynx et/ou l'hypopharynx, un ensemble de sons liés à l'ouverture du sphincter supérieur correspondant au transit du bolus à travers le sphincter supérieur, et un ensemble de sons liés au relâchement laryngé correspondant à la descente et à l'ouverture du larynx quand le bolus a atteint l'œsophage, comme décrit par Sylvain Morinière et al. dans « Origin of the Sound Components During Pharyngeal Swallowing in Normal Subjects », Dysphagia, 2008.

Ces dispositifs colliers peuvent être utilisés pour la détection et l'analyse de déglutitions chez un patient atteint de dysphagie, ou pour l'évaluation des exercices de rééducation à la déglutition. Dans la détection et l'analyse de déglutitions, un utilisateur porte un dispositif collier pour lequel les signaux sont analysés généralement par un dispositif connecté au collier tel qu'un smartphone ou un autre dispositif électronique spécialisé. Ces dispositifs électroniques proposent un certain niveau de détection de la déglutition mais pourraient être améliorés. En effet, le son capté par le microphone peut comprendre des sons environnants induisant alors un bruit menant à une moins bonne détection de la déglutition. De la même façon pour l'accélérométrie, l'accéléromètre peut capter des mouvements ou vibrations non liés à la déglutition mais par exemple aux mouvements du patient, menant là aussi à une moins bonne détection ou caractérisation de la déglutition.

Il existe donc un besoin de pouvoir proposer une meilleure détection de la déglutition en s'abstrayant des bruits provenant de l'environnement.

### RESUME DE L'INVENTION

L'invention offre une solution aux problèmes évoqués précédemment, en permettant une meilleure caractérisation de l'activité pharyngolaryngée et en particulier de la déglutition prenant en compte les signaux de différents capteurs de même type placés dans une configuration spatiale prédéterminée.

Un aspect de l'invention concerne un système de caractérisation de l'activité pharyngolaryngée d'un utilisateur, le système comprenant au moins :
- un dispositif de captation comprenant une pluralité de capteurs d'activité pharyngolaryngée, la pluralité de capteurs comprenant au moins deux capteurs d'activité pharyngolaryngée de même type placés selon une configuration spatiale prédéterminée, chaque capteur étant configuré pour mesurer au moins un signal d'activité pharyngolaryngée de l'utilisateur lors d'au moins une activité pharyngolaryngée de l'utilisateur,
- au moins un processeur configuré pour réaliser une caractérisation de la au moins une activité pharyngolaryngée de l'utilisateur, la caractérisation étant basée sur chacun des signaux d'activité pharyngolaryngée provenant de chacun des capteurs de la pluralité de capteurs d'activité pharyngolaryngée.

Grâce à l'invention, il est possible d'étudier l'activité pharyngolaryngée d'un utilisateur et en particulier la déglutition de manière plus fiable que dans l'état de l'art. En effet, selon l'invention, plusieurs signaux provenant de capteurs de même type sont utilisés afin de fiabiliser la caractérisation des activités pharyngolaryngées détectées. Ainsi, la caractérisation n'est plus seulement temporelle mais aussi spatiale. En prenant en compte les signaux de même type placés selon une configuration spatiale prédéterminée, l'invention permet aussi de supprimer ou au moins de limiter les bruits captés pouvant résulter en une mauvaise caractérisation.

L'invention utilise avantageusement une caractérisation prenant en compte pour une même activité pharyngolaryngée plusieurs signaux captés à différents emplacements de l'utilisateur, les plusieurs signaux étant de même type. Les différents signaux de même type pris en compte dans la caractérisation permettent un recoupement des informations. Ainsi, par exemple une déglutition peut être classée comme déglutition « correcte » ou « incorrecte » de manière plus fine que dans l'état de l'art, en prenant en compte des paramètres spatiaux grâce à la configuration spatiale prédéterminée des capteurs de même type.

Outre les caractéristiques qui viennent d'être évoquées dans le paragraphe précédent, le système de caractérisation selon un aspect de l'invention peut présenter une ou plusieurs caractéristiques complémentaires parmi les suivantes, considérées individuellement ou selon toutes les combinaisons techniquement possibles :
- le dispositif de captation est destiné à être placé au niveau du cou de l'utilisateur.
- le processeur réalise la caractérisation en mettant en œuvre une méthode basée sur un apprentissage automatique.
- les capteurs de la pluralité de capteurs sont de type accéléromètre pour détecter au moins une vibration lié à l'activité pharyngolaryngée ou microphone pour détecter au moins un son d'activité pharyngolaryngée.
- la pluralité de capteurs comprend en outre au moins un capteur d'un autre type que les type des au moins deux capteurs de même type.
- le système comprend au moins une bande de matériau souple à laquelle est maintenue la pluralité de capteurs.
- que le système comprend une pluralité de bandes de matériau souple, chaque bande de matériau souple étant adhésive et formant un patch, au moins un capteur de la pluralité de capteurs étant maintenu à chaque patch.

Un autre aspect de l'invention concerne un procédé de caractérisation de l'activité pharyngolaryngée mis en œuvre par un système de caractérisation de l'activité pharyngolaryngée selon l'une des revendications précédentes, le procédé comprenant les étapes de :
- Mesure d'au moins un signal d'activité pharyngolaryngée par chaque capteur d'activité pharyngolaryngée de même type de la pluralité de capteurs d'activité pharyngolaryngée, les signaux d'activité pharyngolaryngée représentant au moins une activité pharyngolaryngée ;
- Caractérisation, par le processeur du système, de la au moins une activité pharyngolaryngée, la caractérisation étant basée sur le au moins un signal d'activité pharyngolaryngée mesurée par chaque capteur.

Outre les caractéristiques qui viennent d'être évoquées dans le paragraphe précédent, le procédé de caractérisation selon un aspect de l'invention peut présenter une ou plusieurs caractéristiques complémentaires parmi les suivantes, considérées individuellement ou selon toutes les combinaisons techniquement possibles :
- l'activité pharyngolaryngée est une activité pharyngolaryngée parmi les activités d'expectoration, de phonation, de respiration, de déglutition.
- l'activité pharyngolaryngée est une déglutition et la caractérisation comprend la classification de la déglutition au moins dans une classe représentative d'une déglutition correcte ou dans une classe représentative d'une déglutition incorrecte.
- le procédé de caractérisation comprend préalablement au moins une étape d'entraînement à partir d'une pluralité de signaux d'activité pharyngolaryngée d'entraînement, la pluralité de signaux d'activité pharyngolaryngée d'entraînement représentant au moins deux activités pharyngolaryngées d'entraînement d'un utilisateur d'entraînement et comprenant, pour chaque activité pharyngolaryngée d'entraînement, une pluralité de signaux de même type acquis à des emplacements de l'utilisateur selon la configuration spatiale prédéterminée.

Un autre aspect de l'invention concerne un produit-programme d'ordinateur comprenant des instructions qui, lorsque le programme est exécuté par un ordinateur, conduisent celui-ci à mettre en œuvre les étapes du procédé de caractérisation de l'activité pharyngolaryngée selon l'invention.

Encore un autre aspect de l'invention concerne un support d'enregistrement lisible par ordinateur comprenant des instructions qui, lorsqu'elles sont exécutées par un ordinateur, conduisent celui-ci à mettre en œuvre les étapes du procédé de caractérisation de l'activité pharyngolaryngée selon l'invention.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 montre une représentation schématique d'un système de caractérisation de l'activité pharyngolaryngée selon un mode de réalisation de l'invention,
- La figure 2 montre une représentation schématique d'une implémentation d'un système de caractérisation de l'activité pharyngolaryngée selon l'invention,
- La figure 3 montre une représentation schématique d'une autre implémentation d'un système de caractérisation de l'activité pharyngolaryngée selon l'invention,
- Les figures 4a, 4b, 4c et 4d montrent des représentations schématiques de configurations spatiales de capteurs de même type dans un système selon l'invention,
- La figure 5 montre une représentation schématique d'un procédé de caractérisation de l'activité pharyngolaryngée selon l'invention.

### DESCRIPTION DETAILLEE

Les figures sont présentées à titre indicatif et nullement limitatif de l'invention.

Sauf précision contraire, un même élément apparaissant sur des figures différentes présente une référence unique.

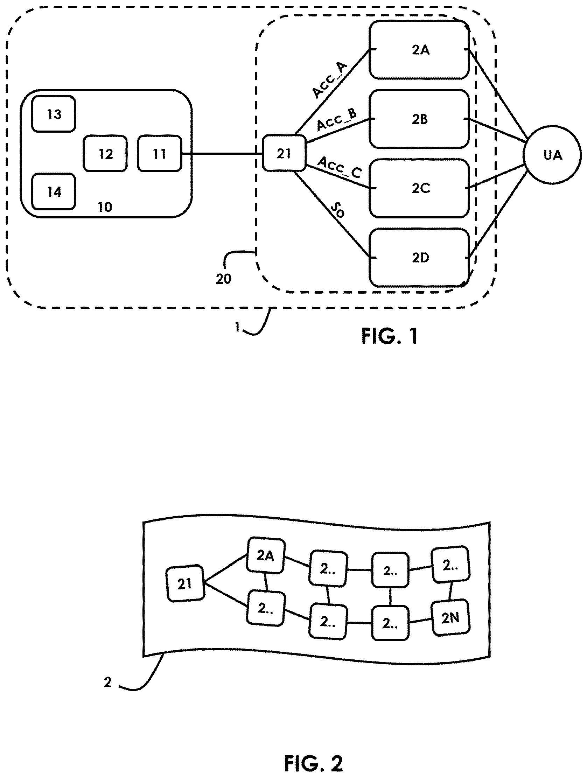

[Fig. 1] montre une représentation schématique d'un système de caractérisation de l'activité pharyngolaryngée selon l'invention.

Le système 1 de caractérisation de l'activité pharyngolaryngée représenté à la [Fig. 1] comprend un dispositif de traitement 10 et un dispositif de captation de l'activité pharyngolaryngée 20 comprenant une pluralité de capteurs 2A, 2B, 2C et 2D.

Bien que la Figure 1 représente un dispositif de captation de l'activité pharyngolaryngée 20 comprenant quatre capteurs 2A à 2D, l'invention n'est bien entendu pas limitée à quatre capteurs et couvre tout dispositif 20 comprenant une pluralité de capteurs de même type, c'est-à-dire au moins deux capteurs de même type. On entend par « capteurs de même type » des capteurs permettant d'acquérir le même type de signal. On entend par type de signal un type parmi un signal de vibration lié à l'activité pharyngolaryngée, le son d'activité pharyngolaryngée et l'activité musculaire d'activité pharyngolaryngée. Ainsi, lorsque les capteurs 2A à 2C sont des accéléromètres, ils sont de même type. Lorsque les capteurs 2A à 2C sont des microphones, ils sont de même type. On entend par « microphone » tout type de capteur permettant de convertir un signal acoustique en signal électrique. Lorsque les capteurs 2A à 2C sont des électromyogrammes, c'est-à-dire des capteurs de signal musculaire, ils sont de même type.

Le capteur 2D représenté à la Figure 1 est un capteur d'activité pharyngolaryngée d'un autre type que les capteurs 2A à 2C. Comme représenté à la Figure 1, le capteur 2D est un microphone captant un signal de son d'activité pharyngolaryngée So, tandis que les capteurs respectivement 2A à 2C sont des accéléromètres captant chacun au moins un signal vibratoire et/ou de mouvement de l'activité pharyngolaryngée respectivement Acc_A à Acc_C. Un signal vibratoire et/ou de mouvement de l'activité pharyngolaryngée est par exemple un signal de mouvement laryngé correspondant à une déglutition ou tout mouvement permettant de caractériser une activité pharyngolaryngée, c'est-à-dire d'attribuer à une activité pharyngolaryngée une étiquette, par exemple « déglutition », « phonation », « respiration » ou « expectoration ».

Les capteurs 2A à 2D peuvent être tout capteur capable de mesurer un signal d'activité pharyngolaryngée permettant de caractériser une activité pharyngolaryngée de l'utilisateur, l'invention couvrant tout dispositif comprenant une pluralité de capteurs de même type.

Un dispositif de captation de l'activité pharyngolaryngée 20 selon l'invention peut être porté par l'utilisateur UA, par exemple dans le mode de réalisation préférentiel dans lequel le dispositif de captation de l'activité pharyngolaryngée 20 est un dispositif collier. Dans ce cas, les capteurs 2A à 2D sont fixés à une bande de matériau souple de dimension suffisante pour faire le tour du cou de l'utilisateur au niveau de son larynx, ou de manière à pouvoir être fixé au niveau du larynx de l'utilisateur. Une telle bande de matériau souple est représentée à la Figure 2. En outre, les capteurs peuvent être maintenus à l'utilisateur par tout moyen, et en tout endroit de l'utilisateur permettant de capter un signal relatif à une activité pharyngolaryngée.

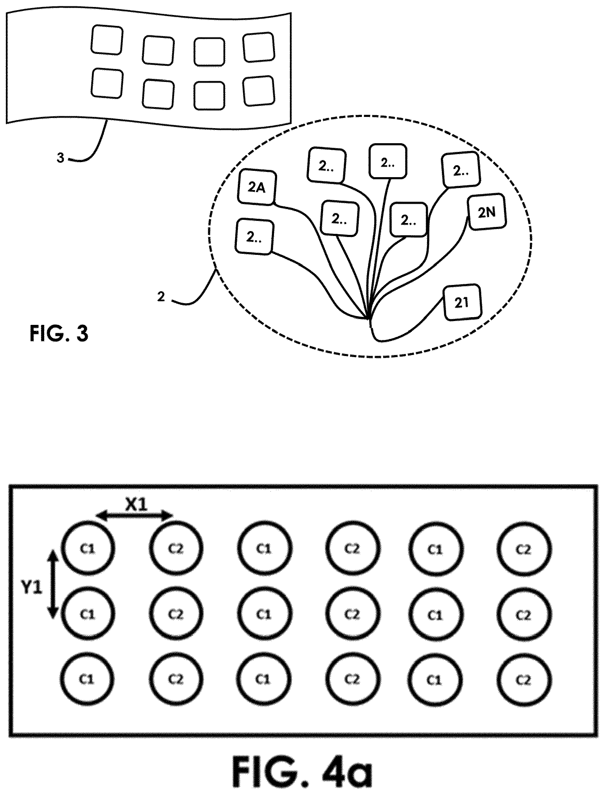

Dans un mode de réalisation alternatif représenté à la Figure 3, les capteurs 2A à 2D peuvent être fixés à une pluralité de patchs, chaque patch ne comprenant qu'un ou plusieurs capteurs, préférentiellement un capteur. Dans ce mode de réalisation alternatif, un guide 2 peut être utilisé avec le système de patchs 3 permettant à un praticien ou à un utilisateur de placer les patchs aux emplacements prédéterminés selon la configuration spatiale prédéterminée selon le guide 2 sur le patient. En fonction du patient et/ou de la condition médicale que l'on cherche à évaluer, plusieurs guides 2 différents peuvent être fournis et utilisés afin de modifier les emplacements prédéterminés et donc les configurations spatiales prédéterminées. Par exemple, un guide peut définir une certaine configuration spatiale prédéterminée de capteurs de même type pour permettre de détecter et/ou caractériser une activité pharyngolaryngée au niveau du larynx, quand un autre guide peut définir une autre configuration spatiale prédéterminée de capteurs de même type permettant de détecter et/ou caractériser une activité pharyngolaryngée au niveau de l'arrière du cou ou du thorax.

Comme représenté aux Figures 2 et 3, les capteurs 2A à 2N sont placés selon une configuration spatiale prédéterminée. C'est-à-dire que les capteurs 2A à 2N sont placés à des emplacements prédéterminés. Par exemple, les capteurs 2A à 2N peuvent être placés le long d'un axe faisant le tour du cou au niveau du larynx de l'utilisateur UA espacés d'une distance prédéterminée. Les capteurs 2A à 2N peuvent aussi être placés dans de zones d'intérêt toujours au niveau du larynx de l'utilisateur UA, au niveau de l'arrière du cou de l'utilisateur UA, au niveau du thorax de l'utilisateur UA, ou une combinaison de ces emplacements. Les capteurs 2A à 2N comprennent au moins deux capteurs de même type placés selon une configuration spatiale prédéterminée et peuvent en outre comprendre plusieurs capteurs d'autres types. Des exemples de configurations spatiales sont représentés aux Figures 4a à 4d.

La Figure 4a représente une configuration spatiale dans laquelle des capteurs de type C1 et des capteurs de type C2 sont placés en grille. La configuration spatiale est ainsi une grille de trois lignes par six colonnes, les lignes étant espacées d'un pas Y1 et les colonnes d'un pas X1. Dans la configuration spatiale représentée à la Figure 4a, chaque colonne ne comprend que des capteurs de même type, et le type de capteur compris dans chaque colonne alterne une colonne sur deux : ainsi, une première colonne comprend des capteurs de type C1, par exemple des accéléromètres, quand la colonne suivante comprend des capteurs de type C2, par exemple des capteurs de son. Le pas X1 est par exemple de 20 mm et le pas Y1 est par exemple de 10 mm. Une configuration spatiale en grille permet par exemple de capturer l'activité pharyngolaryngée au niveau du larynx de l'utilisateur UA ou tout le tour du cou de l'utilisateur UA.

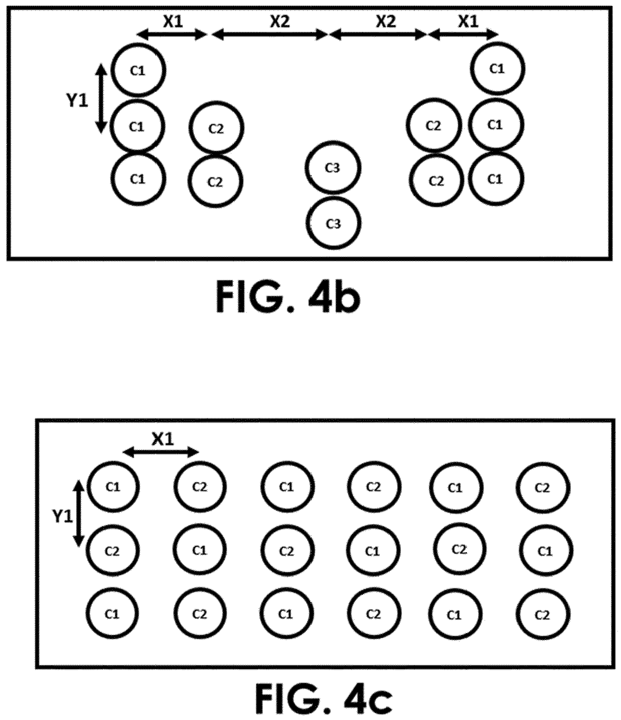

La Figure 4b représente une configuration spatiale dans laquelle des capteurs de trois types différents C1 à C3, dont plusieurs capteurs pour chaque type, sont placés en V. Cela permet par exemple de capturer, avec un premier type C3 de capteurs tels que des microphones, le son laryngé au niveau du larynx de l'utilisateur UA, avec un type C1 de capteurs tels que des accéléromètres le mouvement d'activité laryngée au niveau des muscles sterno-cléido-mastoïdiens et avec un type C2 de capteurs tels que des microphones le son du passage du bolus de part et d'autre de la zone laryngée. Les pas Y1 et X1 peuvent par exemple être les mêmes qu'à la Figure 4a, et le pas X2 peut par exemple être de 25mm.

La Figure 4c représente une autre configuration spatiale de type grille, mais, à la différence de la Figure 4a, chaque colonne de la configuration spatiale de la Figure 4c comprend plusieurs types de capteurs C1 et C2 alternés. Ainsi, les types de capteurs C1 et C2 sont alternés sur les lignes et sur les colonnes pour avoir une répartition semblable des capteurs de type C1 et des capteurs de type C2. Les pas X1 et Y1 peuvent par exemple être les mêmes qu'à la Figure 4a.

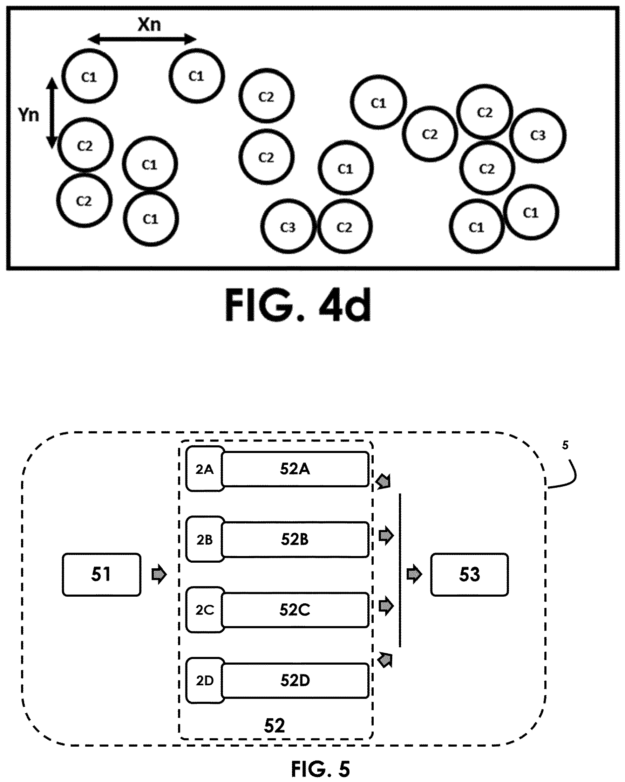

La Figure 4d montre une configuration spatiale à pas aléatoire. Une telle configuration spatiale peut être très spécifique à un type d'activité pharyngolaryngée caractérisée, les capteurs n'étant pas espacés d'un pas constant ou générique.

En référence à la Figure 1, le dispositif de captation de l'activité pharyngolaryngée 20 peut comprendre une interface réseau 21, configurée pour envoyer les signaux d'activité pharyngolaryngée mesurés par les capteurs d'activité pharyngolaryngée 2A à 2D à travers un réseau ou directement via une connexion filaire ou sans fil au dispositif de traitement 10 du système 1. Cette transmission peut être réalisée en Bluetooth^{®}, en Wi-Fi^{®}, ou selon tout autre protocole de transfert de données sans-fil ou filaire, préférentiellement sans-fil. L'interface réseau 21 peut en outre permettre la réception de données en étant configurée pour recevoir des données. Cela peut permettre de réaliser des mises à jour, des réglages ou du pilotage à distance des capteurs de détection d'activité pharyngolaryngée ou d'un microprocesseur (non représenté) du dispositif 20.

Le système 1 de caractérisation de l'activité pharyngolaryngée de l'utilisateur UA comprend en outre un dispositif de traitement 10. Le dispositif de traitement 10 met en œuvre une méthode de caractérisation basée sur un apprentissage automatique, c'est-à-dire par exemple qu'il utilise un ou plusieurs modèles entraînés par apprentissage automatique. Une telle méthode de caractérisation peut par exemple être un réseau de neurones entraîné, une machine à vecteur de support (« SVM »), ou toute autre méthode permettant une caractérisation basée sur un apprentissage automatique.

Le dispositif de traitement 10 selon l'invention, et comme représenté à la [Fig. 1], comprend une interface réseau 11, un processeur 12, un module de stockage 13 et peut comprendre en outre un module d'affichage 14.

Le dispositif de traitement 10 comprend une interface réseau 11 configurée pour recevoir les signaux d'activité pharyngolaryngée mesurés par les capteurs d'activité pharyngolaryngée 2A à 2D à travers un réseau ou directement via une connexion filaire ou sans fil au dispositif de captation de l'activité pharyngolaryngée 20. L'interface réseau 11 peut en outre permettre l'envoi de données à l'interface réseau 21 du dispositif de captation de l'activité pharyngolaryngée 20. Cela peut permettre de réaliser des mises à jour à distance des capteurs de détection de la déglutition ou d'un microprocesseur (non représenté) du dispositif 20.

Le dispositif de traitement 10 comprend au moins un processeur 12. Le processeur 12 est configuré pour réaliser une caractérisation de chacun des signaux d'activité pharyngolaryngée reçus. On entend par « caractérisation » le fait de donner au moins un attribut à un signal capté, par exemple une classe correspondant à une déglutition correcte ou une classe correspondant à une déglutition incorrecte ou une classe parmi les classes correspondant à une déglutition, une expectoration, une phonation, une respiration, ou par exemple une mesure d'intensité.

Cette caractérisation peut être réalisée de manière connue de l'homme du métier par un algorithme d'apprentissage automatique utilisant un modèle statistique ou encore un réseau de neurones. Les signaux issus des capteurs de même type 2A à 2C permettent au modèle de caractérisation d'être plus précis et plus fiable dans ses prises de décision que les modèles de caractérisation de l'art antérieur ne prenant en compte qu'un signal de même type par exemple pour caractériser un signal de déglutition comme étant « incorrect », c'est-à-dire représentatif d'une dysphagie de l'utilisateur étudié, par exemple une déglutition présentant un risque de fausse-route ou d'aspiration, ou «correct», c'est-à-dire représentatif d'une déglutition non caractéristique d'une dysphagie de l'utilisateur étudié.

Le modèle de l'algorithme d'apprentissage automatique a préalablement été entraîné avec une pluralité de signaux d'activité pharyngolaryngée d'entraînement, la pluralité de signaux d'activité pharyngolaryngée d'entraînement représentant au moins une activité pharyngolaryngée d'entraînement d'un utilisateur d'entraînement et comprenant, pour chaque activité pharyngolaryngée d'entraînement, une pluralité de signaux de même type acquis aux mêmes emplacements du larynx de l'utilisateur UA que lors de l'utilisation. Les signaux d'entraînement peuvent être annotés, afin de réaliser un apprentissage automatique supervisé, ou non annotés, afin de réaliser un apprentissage automatique non supervisé. Ainsi, le modèle de caractérisation est entraîné à caractériser les mêmes types de données que les données d'activité pharyngolaryngée captées. En outre, le modèle de caractérisation est entraîné à caractériser, pour une activité pharyngolaryngée d'entraînement, dans la même configuration spatiale le même nombre de signaux d'entraînement de même type que le nombre de signaux de même type captés pour une activité pharyngolaryngée en utilisation courante après apprentissage.

Une fois une activité pharyngolaryngée caractérisée, les signaux représentatifs de l'activité pharyngolaryngée issus des capteurs 2A à 2D peuvent être stockés par un module de stockage 13 du dispositif de traitement 10. Les signaux représentatifs de l'activité pharyngolaryngée caractérisée peuvent être stockés conjointement à l'attribut caractérisant l'activité pharyngolaryngée attribué par le processeur 12.

Le module de stockage 13 du dispositif de traitement 10 peut être un module « temporaire », c'est-à-dire un module de stockage pendant une durée donnée, par exemple une semaine, avant transmission des données stockées à une base de données avec plus de capacité de stockage. Cette transmission peut être réalisée à travers un réseau de communication.

En outre, le dispositif de traitement 10 peut comprendre un module d'affichage 14, configuré pour afficher les signaux d'activité pharyngolaryngée stockés de même type associés à une activité pharyngolaryngée, le résultat de la caractérisation des signaux d'activité pharyngolaryngée de même type stockés ou des représentations statistiques de ces résultats. Le module d'affichage 14 peut être un écran ou tout autre type de dispositif permettant d'afficher des données à un utilisateur.

Dans un mode de réalisation, le dispositif de traitement 10 est compris dans le même dispositif que le dispositif de captation de l'activité pharyngolaryngée 20. Dans ce cas, le dispositif de traitement 10 peut ne pas comprendre d'affichage 14. Dans un autre mode de réalisation, le dispositif de traitement 10 est à distance du dispositif de captation de l'activité pharyngolaryngée 20 et ne fait pas partie du même dispositif.

La [Fig. 5] montre une représentation schématique d'un procédé de caractérisation de l'activité pharyngolaryngée selon l'invention.

Le procédé 5 de caractérisation de l'activité pharyngolaryngée selon l'invention est mis en œuvre par le système 1 de caractérisation de l'activité pharyngolaryngée selon l'invention tel que décrit précédemment et aux Figures 1 à 4.

Le procédé 5 comprend une première étape 51 d'entraînement à partir d'une pluralité de signaux d'activité pharyngolaryngée d'entraînement. Cette première étape d'entraînement 51 comprend l'estimation d'un modèle à partir des signaux d'activité pharyngolaryngée d'entraînement. La pluralité de signaux d'activité pharyngolaryngée d'entraînement représente au moins deux activités pharyngolaryngées d'entraînement d'un utilisateur d'entraînement et comprend, pour chaque activité pharyngolaryngée d'entraînement, une pluralité de signaux de même type acquis aux mêmes emplacements selon la configuration spatiale prédéterminée de l'utilisateur d'entraînement que les emplacements selon la configuration spatiale prédéterminée ensuite utilisés sur l'utilisateur UA. Lorsque l'on mentionne qu'un ou plusieurs signaux représente(nt) une activité pharyngolaryngée, on entend que le ou les signaux ont été capturés par les capteurs 2A à 2D pendant ladite activité pharyngolaryngée, permettant de rapporter certaines des caractéristiques de l'activité pharyngolaryngée.

Le procédé 5 comprend une seconde étape 52 de mesure d'au moins un signal d'activité pharyngolaryngée par chaque capteur d'activité pharyngolaryngée de même type 2A à 2C. Cette étape de mesure est réalisée au moins par chacun des capteurs de même type 2A à 2C et peut aussi être réalisée par au moins un autre capteur d'un autre type 2D comme représenté à la Figure 5.

Le procédé 5 de caractérisation de l'activité pharyngolaryngée comprend une troisième étape 53 de caractérisation, par le dispositif de traitement 10 et en particulier par le processeur 12 du dispositif de traitement 10, de la au moins une activité pharyngolaryngée. Cette caractérisation peut par exemple comprendre la classification de l'activité pharyngolaryngée dans au moins une classe représentative d'une activité d'expectoration, de phonation, de respiration ou de déglutition. Lors d'une caractérisation d'une déglutition, la caractérisation peut par exemple comprendre la classification de la déglutition dans au moins une classe représentative d'une déglutition correcte ou dans une classe représentative d'une déglutition incorrecte. En outre, la caractérisation peut comprendre la spatialisation de l'activité pharyngolaryngée, par exemple pour réaliser une représentation ou pour localiser le bolus durant une déglutition.

## Revendications

1. Système de caractérisation de l'activité pharyngolaryngée d'un utilisateur, le système comprenant au moins :
- un dispositif de captation comprenant une pluralité de capteurs d'activité pharyngolaryngée, la pluralité de capteurs comprenant au moins deux capteurs d'activité pharyngolaryngée de même type placés selon une configuration spatiale prédéterminée, chaque capteur étant configuré pour mesurer au moins un signal d'activité pharyngolaryngée de l'utilisateur lors d'au moins une activité pharyngolaryngée de l'utilisateur,
- au moins un processeur configuré pour réaliser une caractérisation de la au moins une activité pharyngolaryngée de l'utilisateur, la caractérisation étant basée sur chacun des signaux d'activité pharyngolaryngée provenant de chacun des capteurs de la pluralité de capteurs d'activité pharyngolaryngée.

2. Système de caractérisation de l'activité pharyngolaryngée selon la revendication précédente **caractérisé en ce que** le dispositif de captation est destiné à être placé au niveau du cou de l'utilisateur.

3. Système de caractérisation de l'activité pharyngolaryngée selon l'une des revendications précédentes **caractérisé en ce que** le processeur réalise la caractérisation en mettant en œuvre une méthode basée sur un apprentissage automatique.

4. Système de caractérisation de l'activité pharyngolaryngée selon l'une des revendications précédentes **caractérisé en ce que** les capteurs de la pluralité de capteurs sont de type accéléromètre pour détecter au moins un mouvement d'activité pharyngolaryngée ou microphone pour détecter au moins un son d'activité pharyngolaryngée.

5. Système de caractérisation de l'activité pharyngolaryngée selon l'une des revendications précédentes **caractérisé en ce que** la pluralité de capteurs comprend en outre au moins un capteur d'un autre type que les type des au moins deux capteurs de même type.

6. Système de caractérisation de l'activité pharyngolaryngée selon l'une des revendications précédentes **caractérisé en ce que** le système comprend au moins une bande de matériau souple à laquelle est maintenue la pluralité de capteurs.

7. Système de caractérisation de l'activité pharyngolaryngée selon la revendication précédente **caractérisé en ce que** le système comprend une pluralité de bandes de matériau souple, chaque bande de matériau souple étant adhésive et formant un patch, au moins un capteur de la pluralité de capteurs étant maintenu à chaque patch.

8. Procédé de caractérisation de l'activité pharyngolaryngée mis en œuvre par un système de caractérisation de l'activité pharyngolaryngée selon l'une des revendications précédentes, le procédé comprenant les étapes de :
- Mesure d'au moins un signal d'activité pharyngolaryngée par chaque capteur d'activité pharyngolaryngée de même type de la pluralité de capteurs d'activité pharyngolaryngée, les signaux d'activité pharyngolaryngée représentant au moins une activité pharyngolaryngée ;
- Caractérisation, par le processeur du système, de la au moins une activité pharyngolaryngée, la caractérisation étant basée sur le au moins un signal d'activité pharyngolaryngée mesurée par chaque capteur.

9. Procédé de caractérisation de l'activité pharyngolaryngée selon la revendication précédente **caractérisé en ce que** l'activité pharyngolaryngée est une activité pharyngolaryngée parmi les activités d'expectoration, de phonation, de respiration, de déglutition.

10. Procédé de caractérisation de l'activité pharyngolaryngée selon la revendication précédente **caractérisé en ce que** l'activité pharyngolaryngée est une déglutition et **en ce que** la caractérisation comprend la classification de la déglutition au moins dans une classe représentative d'une déglutition « correcte » ou dans une classe représentative d'une déglutition « incorrecte ».

11. Procédé de caractérisation de l'activité pharyngolaryngée d'un utilisateur selon la revendication précédente **caractérisé en ce qu'**il comprend préalablement au moins une étape d'entraînement à partir d'une pluralité de signaux d'activité pharyngolaryngée d'entraînement, la pluralité de signaux d'activité pharyngolaryngée d'entraînement représentant au moins deux activités pharyngolaryngées d'entraînement d'un utilisateur d'entraînement et comprenant, pour chaque activité pharyngolaryngée d'entraînement, une pluralité de signaux de même type acquis à des emplacements de l'utilisateur selon la configuration spatiale prédéterminée.

12. Produit-programme d'ordinateur comprenant des instructions qui, lorsque le programme est exécuté par un ordinateur, conduisent celui-ci à mettre en œuvre les étapes du procédé de caractérisation de l'activité pharyngolaryngée selon l'une quelconque des revendications 8 à 11.

13. Support d'enregistrement lisible par ordinateur comprenant des instructions qui, lorsqu'elles sont exécutées par un ordinateur, conduisent celui-ci à mettre en œuvre les étapes du procédé de caractérisation de l'activité pharyngolaryngée selon l'une quelconque des revendications 8 à 11.
